# EUROPÄISCHE PATENTSCHRIFT

(11) **EP 0 602 585 B1**
(45) Veröffentlichungstag und Bekanntmachung des Hinweises auf die Patenterteilung: **16.10.1996**
(21) Anmeldenummer: 93120095.0
(22) Anmeldetag: 14.12.1993
(51) Int. Cl.: A61K 9/08, A61K 31/72

(54) **Dialyselösung für die Peritonealdialyse**
Dialysis solution for a peritoneal dialysis
Solution de dialyse pour la dialyse péritonéale

(30) Priorität: 18.12.1992 DE 4242926
(43) Veröffentlichungstag der Anmeldung: 22.06.1994
(73) Patentinhaber: Fresenius AG, D-61350 Bad Homburg v.d.H (DE)
(72) Erfinder: Sommermeyer, Klaus, Dr., D-61191 Rosbach v.d.H. (DE); Passlick-Deetjen, Jutta, Dr., D-35392 Giessen (DE)
(74) Vertreter: Fuchs, Luderschmidt & Partner Patentanwälte

(56) Entgegenhaltungen:
- EP-A- 0 402 724
- DE-A- 3 428 201
- NEPHRON Bd. 61, Nr. 1 , 1992 Seite 120 N.GRETZ ET AL. 'HES as an osmotic agent for continuous ambulatory peritoneal dialysis solutions'
- KLINISCHE WOCHENSCHRIFT Bd. 62 , 1984 Seiten 862 - 866 U.PFEIFER ET AL. 'Ascites als Komplikation hepatischer Speicherung von Hydroxyethylstärke (HES) bei Langzeitdialyse'
- CAPD - A Decade of Experience. Contrib. Nephrol. Basel, Karger, Band 89, 1991, Seiten 119-127

## Beschreibung

Gegenstand der vorliegenden Erfindung sind Dialyselösungen für die Peritonealdialyse, die Hydroxyethylstärke als osmotisch wirksame Substanz, Elektrolyte und/oder übliche Zusätze enthalten.

In Lösungen für die Peritonealdialyse, zum Beispiel die kontinuierliche ambulante Peritonealdialyse (CAPD) oder die kontinuierliche Cycling-Peritonealdialyse (CCPD) wird üblicherweise Glucose als osmotische Trägersubstanz oder osmotisch wirksame Substanz eingesetzt. Solche Lösungen besitzen jedoch den Nachteil, daß die Glucose als osmotisch wirksame Substanz stark resorbiert wird, wobei der Glucosespiegel erhöht wird, was insbesondere dann sehr nachteilig ist, wenn Störungen des Zuckerstoffwechsels vorliegen.

Daher bestehen schon seit längerer Zeit Bestrebungen, in den Dialyselösungen für die Peritonealdialyse die Glucose durch andere Stoffe zu ersetzen, die die bei Verwendung von Glucose als osmotisch wirksame Substanz auftretenden Nachteile nicht aufweisen, d.h. bei denen keine unerwünschte Resorption auftritt, und mit denen auch eine längere Verweilzeit möglich ist und damit ein Wechsel lediglich in größeren Zeitabschnitten erforderlich ist. Als Ersatzstoffe für die Glucose wurden Polyanionen und Glycerin in die engere Wahl gezogen. Polyanionen besitzen jedoch den Nachteil, daß durch sie Veränderungen in der Membranporengröße auftreten können, die sich auf die Dialyse negativ auswirken. Die Arbeiten mit Polyanionen sind bisher nicht über das Stadium der Tierversuche hinausgegangen. Auch gegen die Verwendung von Glycerin bestehen erhebliche Bedenken, da Glycerin eine nephrotoxische Wirkung besitzt und, weil es aufgrund seines niedrigen Molekulargewichts resorbierbar ist, die Restnierenfunktion stören kann.

Gemäß der DE-OS 3428201 wird als osmotisch wirksame Substanz in Dialyselösungen für die Peritonealdialyse eine Hydroxyethylstärke mit einem Molekulargewicht von ≧ 3 x 10⁴ Dalton und einem Substitutionsgrad von 0,25 bis 0,7 vorgeschlagen. Die Verwendung einer solchen Hydroxyethylstärke ist jedoch im Hinblick auf die Problematik der schlechten Verstoffwechselung von eventuell absorbierter Hydroxyethylstärke ungünstig.

Aus der EP-A-0 402 724 sind Hydroxyethylstärken bekannt, die ein mittleres Molekulargewicht von 60 000 bis 600 000, einen Substitutionsgrad MS und DS von 0,15 bis 0,5 und ein Verhältnis der Substitution C2/C6 von 8 bis 20 aufweisen. Beschrieben ist jedoch lediglich der Einsatz als Plasmaexpander, eine Verwendung von Hydroxyethylstärke in der Peritonealdialyse wird nicht offenbart.

Aus Untersuchungen, die in Nephron 1992, 61, 120 genannt sind und in denen der Abbau von Hydroxyethylstärken mit verschiedenen Molekulargewichten in den verschiedenen Organen verglichen wurde, ist jedoch zu entnehmen, daß Hydroxyethylstärke nicht als alternative osmotisch wirksame Substanz in der kontinuierlichen ambulanten Peritonealdialyse geeignet erscheint.

Klin. Wochenschr. (1984), 62 : 862-866 berichtet, daß Plasmaexpander, wie HES, bei Dialysepatienten allenfalls kurzfristig eingesetzt werden dürfen

Es besteht daher nach wie vor ein Bedürfnis nach Dialyselösungen für die Peritonealdialyse, die osmotisch wirksame Substanzen enthalten, mit denen die Nachteile der bekannten Lösungen nicht auftreten.

Aufgabe der vorliegenden Erfindung ist daher, Dialyselösungen für die Peritonealdialyse bereitzustellen, die die Nachteile der bekannten Dialyselösungen nicht aufweisen, sondern eine osmotisch wirksame Substanz enthalten, die wenig bzw. kaum resorbiert wird, leicht verstoffwechselt wird, nicht toxisch ist, für das Peritoneum keine nachteiligen Wirkungen besitzt und gute osmotische Wirksamkeit aufweist.

Überraschend wurde gefunden, daß Dialyselösungen, die dadurch gekennzeichnet sind, daß sie eine Hydroxyethylstärke enthalten, die ein Molekulargewicht Mw von 10.000 bis 150.000, einen Substitutionsgrad MS von 0,10 bis 0,40, einen Substitutionsgrad DS von 0,09 bis 0,35 und ein Verhältnis der Substitution C2/C6 von ≧ 8 aufweist, die Nachteile der bekannten Lösungen nicht aufweisen.

Die in den erfindungsgemäßen Dialyselösungen verwendete Hydroxyethylstärke besitzt vorzugsweise ein Molekulargewicht Mw von 10.000 bis 55.000, insbesondere von 20.000 bis 29.000, zum Beispiel 29.000. Der Substitutionsgrad MS der Hydroxyethylstärke beträgt vorzugsweise 0,10 bis 0,24, insbesondere 0,20 bis 0,24, zum Beispiel 0,23. Vorzugsweise beträgt der Substitutionsgrad DS der verwendeten Hydroxyethylstärke 0,09 bis 0,23, während das Verhältnis C2/C6 der verwendeten Stärke vorzugsweise 8 bis 25 beträgt.

Die Herstellung der erfindungsgemäß eingesetzten Hydroxyethylstärke erfolgt in an sich bekannter Weise durch hydrolytischen und/oder enzymatischem Abbau einer Stärke, insbesondere einer an Amylopektin reichen Stärke, auf ein bestimmtes Molekulargewicht und partielle Verätherung bis zum gewünschten Substitutionsgrad. Zur Herstellung der Dialyselösung kann eine feste, zum Beispiel durch Sprühtrocknung oder Vakuumtrocknung gewonnene Hydroxyethylstärke eingesetzt werden oder können aber auch die nach der Reinigung, insbesondere Diafiltration, erhaltenen Hydrolyselösungen verwendet werden.

Die erfindungsgemäße Dialyselösung enthält die Hydroxyethylstärke vorzugsweise in einer Menge von 3 bis 10 Gew%, bezogen auf die fertige Lösung.

Als Medium für die Dialyselösung kann zum Beispiel jedes für die Peritonealdialyse, insbesondere für die Peritonealdialyse mit Glucose als osmotischer Trägersubstanz übliche Medium verwendet werden, z.B. eine Ringer-Lösung.

Neben der Hydroxyethylstärke als osmotisch wirksamer Substanz können die erfindungsgemäßen Lösungen zur Peritonealdialyse noch weitere Substanzen enthalten, insbesondere die neben der osmotisch wirksamen Substanz üblichen Bestandteile von Peritonealdialyse-Lösungen, wie z.B. die Bestandteile der Ringer-Lösung. Eine erfindungsgemäß bevorzugte Dialyselösung enthält zum Beispiel 3 bis 10 Gewichtsprozent Hydroxyethylstärke, 125 bis 150 mMol/l Natriumionen, 90 bis 115 mMol/l Chloridionen, 0,3 bis 1,5 mMol/l Magnesiumionen, 1,0 bis 2,5 mMol/l Calciumionen und 30 bis 45 mMol/l Lactat. Das Lactat kann auch ganz oder teilweise durch Acetat und/oder Bicarbonat ersetzt werden. Die genannten Kationen und Anionen liegen dabei vorzugsweise als Salz der ebenfalls als Bestandteile genannten Gegenionen vor. Es können jedoch auch andere physiologisch verträgliche Kationen bzw. Anionen verwendet werden, insbesondere bei mangelnder Menge an Gegenionen.

Als weitere Bestandteile können die erfindungsgemäßen Dialyselösungen gegebenenfalls eine oder mehrere pharmakologisch aktive Substanzen enthalten, wie sie z.B. auch in bisher üblichen Dialyselösungen verwendet werden. Solche Wirkstoffe sind insbesondere Vasodilatatoren, wie z.B. Natriumnitroprussid, Diuretika, Hormone, wie z.B. Insulin und/oder Vitamine, wie z.B. insbesondere Vitamin E. Die Art und Menge dieser Wirkstoffe richtet sich dabei insbesondere nach dem jeweiligen Einzelfall.

Als weiterer Bestandteil kann Glucose, vorzugsweise in einer Menge von 0,1 bis 4,25 Gew.%, bezogen auf die fertige Lösung, in der erfindungsgemäßen Dialyselösung enthalten sein. Ein Zusatz an Glucose ist insbesondere dann zweckmäßig, wenn mit der Dialyselösung auch eine gewisse Nahrungszufuhr beabsichtigt ist.

Die Menge der neben der Hydroxyethylstärke vorhandenen Bestandteile ist insbesondere vom Gehalt an Hydroxyethylstärke abhängig, wobei sich die Art und Menge der pharmakologisch wirksamen Substanzen insbesondere nach dem jeweiligen Einzelfall richtet.

Die erfindungsgemäße Dialyselösung weist eine niedrige Osmolarität auf und ist vorzugsweise im wesentlichen isoosmolar.

Die erfindungsgemäßen Dialyselösungen können in an sich bekannter Weise durch Auflösen der Bestandteile in destilliertem Wasser, Filtrieren der erhaltenen Lösung und anschließende Sterilisation der Lösung hergestellt werden. Die Bestandteile können dabei gleichzeitig zugesetzt und gelöst werden, es kann jedoch auch stufenweise derart vorgegangen werden, daß man einen oder mehrere Bestandteile nacheinander zusetzt und auflöst. Zum Beispiel können die Hydroxyethylstärke und gegebenenfalls weitere Bestandteile in einer fertigen Ringer-Lösung oder einem anderen, für derartige Dialyselösungen geeignetem Medium aufgelöst werden.

Die Dialyselösung wird in für die Peritonealdialyse geeigneten üblichen bekannten Behältern aufbewahrt und verwendet. Derartige Behälter können sowohl aus starrem (z.B. Glas) oder aber auch aus einem biegsamen Material (z.B. Kunststoffmaterial) und insbesondere aus dünnen zusammenfaltbaren Kunststoffolien bestehen.

Vorteilhafter wird die Herstellung und/oder Sterilisation der Dialyselösung bereits in den zur Aufbewahrung und Verwendung geeigneten Behältern durchgeführt. In verschiedener Hinsicht (Lagerung, Transport, Stabilität der Lösung, Wahl der Dialyselösung je nach Zweckbestimmung) kann es jedoch auch zweckmäßig sein, einige oder alle Bestandteile der Dialyselösung in fester Form, z.B. in lyophilisierter Form, in die Dialysebehälter abzufüllen und erst kurz vor ihrer Verwendung in dem geeigneten Medium, z.B. in destilliertem Wasser oder Ringer-Lösung, aufzulösen.

Die erfindungsgemäßen Dialyselösungen erlauben, verglichen mit den bekannten Dialyselösungen, die Glucose als osmotisch wirksame Substanz enthalten, eine höhere Anwendungsdauer, d.h. die Verweilzeit der Dialyselösung ist erhöht. So ist es möglich, z.B. bei Verwendung der erfindungsgemäßen Dialyselösung die CAPD über einen Zeitraum von 12 Stunden (ohne Wechsel) durchzuführen. Die erfindungsgemäße Dialyselösung ist insbesondere auch für Patienten mit Restfunktion der Niere vorteilhaft. Die Resorption der osmotisch wirksamen Substanz ist erfindungsgemäß deutlich gemindert. Darüber hinaus besteht ein weiterer Vorteil darin, daß die erfindungsgemäß eingesetzte Hydroxyethylstärke leicht abgebaut wird und nicht toxisch ist.

Die nachfolgenden Beispiele dienen der Erläuterung der Erfindung.

### Beispiel 1:

Unter Verwendung der nachfolgend aufgeführten Bestandteile wurde eine erfindungsgemäße Dialyselösung hergestellt (Einwaage/1000 ml):

| | |
|---|---|
| Hydroxyethylstärke | 75,0 g |
| Mw 29.000 | |
| MS 0,23 | |
| DS 0,21 | |
| C2/C6 8,7 | |
| Natriumchlorid | 5,435 g |
| Natrium-L-lactat (50%ige Lösung) | 8,97 g |
| Calciumchlorid x 2H₂O | 0,2573 g |
| Magnesiumchlorid x 6 H₂O | 0,0508 g |
| Wasser für Injektionszwecke | 945 ml |

Die Herstellung der erfindungsgemäßen Dialyselösung erfolgte in folgender Weise:

In 945 ml Wasser für Injektionszwecke wurden in der folgenden Reihenfolge das angegebene Natriumchlorid, das Natrium-L-lactat (50%ige Lösung), das Calciumchlorid, das Magnesiumchlorid und die Hydroxyethylstärke gebracht und unter Rühren gelöst. Nach vollständigem Lösen wurden 0,25 g Aktivkohle (DAB 9) zugegeben und nach 20-minütigem Rühren durch Seitz Filterschichten Supra EK 1P und anschließend über Membranfilter 0,2 µm in den Kühltank filtriert. Der pH-Wert wurde entweder durch Zugabe von 25%iger Salzsäure oder von Natriumhydroxid auf pH 5,8 bis 5,9 eingestellt. Die erhaltene Lösung wurde anschließend durch Membranvorfilter und dann über Membranfilter 0,2 µm filtriert. Danach wurde die Lösung in geeignete Behälter abgefüllt und nach Verschließen der Behälter sterilisiert.

Sterilisation: Stand 121°C, 15 Min.
zuzüglich der Ausgleichszeit.

Die erhaltene Lösung besaß folgende Merkmale:

| | |
|---|---|
| pH-Wert: | 5,0 bis 6,0, |
| Dichte: | 1,032 bis 1,038 |
| Osmolarität: | 2,72 mosmol/l |
| Titrationsacidität: | 0,3-2,0 mmol NaOH/l. |

### Beispiel 2:

Die in Beispiel 1 hergestellte Dialyselösung wurde hinsichtlich ihrer Wirksamkeit als Dialyselösung untersucht.

Zu Vergleichszwecken wurde eine Dialyselösung verwendet, die in gleicher Weise wie die in Beispiel 1 hergestellte Dialyselösung hergestellt wurde und die gleiche Zusammensetzung wie die Lösung von Beispiel 1 aufwies mit der Ausnahme, daß anstelle der in Beispiel 1 eingesetzten Hydroxyethylstärke 42,5 g/l Glucose verwendet wurden.

Die Untersuchungen wurden in üblicher Weise durchgeführt, wobei als Versuchstiere urämische Kaninchen (5/6 nephrektomiert) eingesetzt wurden.

In der Untersuchungsgruppe, in der die Wirksamkeit von HES untersucht wurde, wurden 6 Tiere untersucht und in der Gruppe, in der Glucose als osmotisch wirksame Substanz eingesetzt wurde, wurden 8 Tiere untersucht. Bei diesen Untersuchungen wurde bei 2 Stunden, 4 Stunden, 6 Stunden und 12 Stunden jeweils die Ultrafiltration und die prozentuale Wiederauffindung der jeweils eingesetzten osmotisch wirksamen Substanz (d.h. Hydroxyethylstärke oder Glucose) im Dialysatauslauf bestimmt.

Die Ergebnisse zeigen, daß Hydroxyethylstärke gegenüber Glucose eine ausgezeichnete Ultrafiltration, auch über eine Zeitspanne von 12 Stunden, aufweist. Weiterhin ergibt sich aus den Ergebnissen, daß bei Verwendung von Hydroxyethylstärke nur eine relativ geringe Resorption erfolgt, wobei über eine Zeitspanne von 12 Stunden eine Resorption von lediglich 60-70 % erfolgt, während bei Verwendung von Glucose die Glucose bereits nach relativ kurzer Zeit zu erheblichem Ausmaße resorbiert war und nach einer Zeitspanne von 12 Stunden die gesamte Glucose resorbiert war.

Die Ergebnisse, die bei den Untersuchungen erhalten wurden, sind aus den Tabellen A bis D ersichtlich.

**Tabelle A**

| Ultrafiltration (ml) HES (Therapiephase) | | | |
|---|---|---|---|
| Verweildauer | Tier 1 | Tier 2 | Tier 3 |
| 2 h | 30 | 20 | 20 |
| 4 h | 100 | 55 | 75 |
| 6 h | 200 | 80 | 95 |
| 12 h | 100 | 175 | 105 |

| Verweildauer | Tier 4 | Tier 5 | Tier 6 |
|---|---|---|---|
| 2 h | 130 | 125 | 50 |
| 4 h | 60 | 130 | 105 |
| 6 h | 120 | 145 | 135 |
| 12 h | 140 | 180 | 210 |

**Tabelle B**

| Ultrafiltration (ml) Glucose 4,25 % (Therapiephase) | | | | |
|---|---|---|---|---|
| Verweildauer | Tier 1 | Tier 2 | Tier 3 | Tier 4 |
| 2 h | 100 | 105 | 125 | 55 |
| 4 h | | 100 | 100 | 140 |
| 6 h | 40 | 70 | 70 | 35 |
| 12 h | - 130 | - 80 | - 50 | - 85 |

| Verweildauer | Tier 5 | Tier 6 | Tier 7 | Tier 8 |
|---|---|---|---|---|
| 2 h | - 5 | 90 | 85 | 90 |
| 4 h | - 60 | 60 | 55 | 75 |
| 6 h | - 45 | - 40 | 35 | 0 |
| 12 h | - 160 | - 200 | - 125 | - 150 |

**Tabelle C**

| Prozent. Wiederfindung im Dialysatauslauf HES | | | |
|---|---|---|---|
| Verweildauer | Tier 1 | Tier 2 | Tier 3 |
| 2 h | 65,6 | 61,5 | |
| 4 h | 64,6 | 57,0 | |
| 6 h | 70,4 | 41,0 | |
| 12 h | 23,3 | 39,3 | |

| Verweildauer | Tier 4 | Tier 5 | Tier 6 |
|---|---|---|---|
| 2 h | 73,4 | 42,6 | 74,4 |
| 4 h | 47,7 | 66,2 | 68,7 |
| 6 h | 51,6 | 54,1 | 66,5 |
| 12 h | 38,4 | 38,5 | 48,0 |

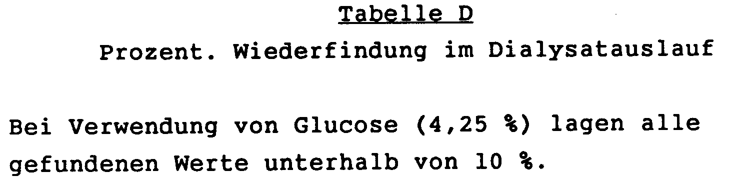

### Beispiel 3:

Beispiel 1 wurde wiederholt mit der Ausnahme, daß anstelle der dort verwendeten Hydroxyethylstärke 75 g einer Hydroxyethylstärke mit folgenden Charakteristika verwendet wurden:
- Mw: 130 000
- MS: 0,4
- DS: 0,35
- C2/C6: 9

## Patentansprüche

1. Verwendung von Hydroxyethylstärke als osmotisch wirksame Substanz in einer Dialyselösung für die Peritonealdialyse, wobei die Lösung neben der Hydroxyethylstärke noch Elektrolyte und/oder übliche Zusätze enthält, dadurch gekennzeichent, daß die Hydroxyethylstärke ein Molekulargewicht Mw von 10.000 bis 150.000, eine Substitution MS von 0,10 bis 0,40, eine Substitution DS von 0,09 bis 0,35 und ein Verhältnis der Substitution von C2/C6 von ≧ 8 aufweist.

2. Verwendung von Hydroxyethylstärke nach Patentanspruch 1, dadurch gekennzeichnet, daß die Hydroxyethylstärke ein Molekulargewicht Mw von 10.000 bis 55.000 besitzt.

3. Verwendung von Hydroxyethylstärke nach Patentanspruch 2, dadurch gekennzeichnet, daß die Hydroxyethylstärke ein Molekulargewicht von 20.000 bis 29.000 besitzt.

4. Verwendung von Hydroxyethylstärke nach Patentanspruch 1 bis 3, dadurch gekennzeichnet, daß die molare Substitution MS der Hydroxyethylstärke 0,10 bis 0,24 beträgt.

5. Verwendung von Hydroxyethylstärke nach Patentanspruch 4, dadurch gekennzeichnet, daß die Substitution MS der Hydroxyethylstärke 0,20 bis 0,24 beträgt.

6. Verwendung von Hydroxyethylstärke nach Patentanspruch 1 bis 5, dadurch gekennzeichnet, daß die Hydroxyethylstärke eine Substitution DS von 0,09 bis 0,23 aufweist.

7. Verwendung von Hydroxyethylstärke nach Patentanspruch 1 bis 6, dadurch gekennzeichnet, daß die Hydroxyethylstärke ein Verhältnis von C2/C6 von 8 bis 25 besitzt.

8. Verwendung von Hydroxyethylstärke nach Patentanspruch 1 bis 7, dadurch gekennzeichnet, daß die Hydroxyethylstärke in einer Konzentration von 3 bis 10 Gewichtsprozent, bezogen auf die fertige Lösung, vorliegt.

9. Verwendung von Hydroxyethylstärke nach Patentanspruch 1 bis 8, dadurch gekennzeichnet, daß sie 3 bis 10 Gewichtsprozent Hydroxyethylstärke, 125 bis 150 mMol/l Natriumionen, 90 bis 115 mMol/l Chloridionen, 0,3 bis 1,5 mMol/l Magnesiumionen, 1,0 bis 2,5 mMol/l Calciumionen und 30 bis 45 mMol/l Lactat und/oder Acetat und/oder Bicarbonat enthält.

10. Verwendung von Hydroxyethylstärke nach Patentanspruch 1 bis 9, dadurch gekennzeichnet, daß sie als weiteren Bestandteil 0,1 bis 4,25 Gewichtsprozent, bezogen auf die fertige Lösung, Glucose enthält.

11. Dialyselösung für die Peritonealdialyse, enthaltend Hydroxyethylstärke als osmotisch wirksame Substanz, Elekrolyte und/oder übliche Zusätze, dadurch gekennzeichnet, daß die Hydroxyethylstärke ein Molekulargewicht Mw von 10.000 bis 55.000, eine Substitution MS von 0,10 bis 0,40, eine Substitution DS von 0,09 bis 0,35 und ein Verhältnis der Substitution von C2/C6 von ≧ 8 aufweist.

12. Dialyselösung nach Patentanspruch 11, dadurch gekennzeichnet, daß die Hydroxyethylstärke ein Molekulargewicht von 20.000 bis 29.000 besitzt.

13. Dialyselösung nach Patentanspruch 11 und 12, dadurch gekennzeichnet, daß die molare Substitution MS der Hydroxyethylstärke 0,10 bis 0,24 beträgt.

14. Dialyselösung nach Patentanspruch 13, dadurch gekennzeichnet, daß die Substitution MS der Hydroxyethylstärke 0,20 bis 0,24 beträgt.

15. Dialyselösung nach Patentanspruch 11 bis 14, dadurch gekennzeichnet, daß die Hydroxyethylstärke eine Substitution DS von 0,09 bis 0,23 aufweist.

16. Dialyselösung nach Patentanspruch 11 bis 15, dadurch gekennzeichnet, daß die Hydroxyethylstärke ein Verhältnis von C2/C6 von 8 bis 25 besitzt.

17. Dialyselösung nach Patentanspruch 11 bis 16, dadurch gekennzeichnet, daß die Hydroxyethylstärke in einer Konzentration von 3 bis 10 Gewichtsprozent, bezogen auf die fertige Lösung, vorliegt.

18. Dialyselösung nach Patentanspruch 11 bis 17, dadurch gekennzeichnet, daß sie 3 bis 10 Gewichtsprozent Hydroxyethylstärke, 125 bis 150 mMol/l Natriumionen, 90 bis 115 mMol/l Chloridionen, 0,3 bis 1,5 mMol/l Magnesiumionen, 1,0 bis 2,5 mMol/l Calciumionen und 30 bis 45 mMol/l Lactat und/oder Acetat und/oder Bicarbonat enthält.

19. Dialyselösung nach Patentanspruch 11 bis 18, dadurch gekennzeichnet, daß sie als weiteren Bestandteil 0,1 bis 4,25 Gewichtsprozent, bezogen auf die fertige Lösung, Glucose enthält.

## Claims

1. Utilisation of hydroxyethyl starch as the osmotically-active substance in a dialysis solution for peritoneal dialysis, where the solution contains electrolytes and/or conventional additives along with the hydroxyethyl starch, characterized in that the hydroxyethyl starch has a molecular weight Mw in the range from 10,000 to 150,000, a degree of substitution MS in the range from 0.10 to 0.40, a degree of substitution DS in the range from 0.09 to 0.35 and a substitution ratio C2/C6 ≧ 8.

2. The utilisation of hydroxyethyl starch according to Claim 1, characterized in that the hydroxyethyl starch has a molecular weight Mw in the range from 10,000 to 55,000.

3. The utilisation of hydroxyethyl starch according to Claim 2, characterized in that the hydroxyethyl starch has a molecular weight in the range from 20,00 to 29,000.

4. The utilisation of hydroxyethyl starch according to Claims 1 to 3, characterized in that the molar substitution MS of the hydroxyethyl starch has a value in the range from 0.10 to 0.24.

5. The utilisation of hydroxyethyl starch according to Claim 4, characterized in that the substitution MS of the hydroxyethyl starch has a value in the range from 0.20 to 0.24.

6. The utilisation of hydroxyethyl starch according to Claims 1 to 5, characterized in that substitution DS of the hydroxyethyl starch has a value in the range from 0.09 to 0.23.

7. The utilisation of hydroxyethyl starch according to Claims 1 to 6, characterized in that the hydroxyethyl starch has a ratio C2/C6 in the range from 8 to 25.

8. The utilisation of hydroxyethyl starch according to Claims 1 to 7, characterized in that the hydroxyethyl starch is present in a concentration in the range from 3 to 10 percent by weight, based on the final solution.

9. The utilisation of hydroxyethyl starch according to Claims 1 to 8, characterized in that it contains from 3 to 10 percent by weight of hydroxyethyl starch, 125 to 150 mMol/litre of sodium ions, 90 to 115 mMol/litre of chloride ions, 0.3 to 1.5 mMol/litre of magnesium ions, 1.0 to 2.5 mMol/litre of calcium ions and 30 to 45 mMol/litre of lactate and/or acetate and/or bicarbonate.

10. The utilisation of hydroxyethyl starch according to Claims 1 to 9, characterized in that it also contains, as additional ingredient, from 0.1 to 4.5 percent by weight of glucose, based on the final solution.

11. Dialysis solution for peritoneal dialysis containing hydroxyethyl starch as the osmotically-active substance, electrolytes and/or conventional additives, characterized in that the hydroxyethyl starch has a molecular weigth Mw in the range from 10,000 to 55,000, a degree of substitution MS in the range from 0.10 to 0.40, a degree of substitution DS in the range from 0.09 to 0.35 and a substitution ratio C2/C6 ≧ 8.

12. The dialysis solution according to Claim 11, characterized in that the hydroxyethyl starch has a molecular weigth Mw in the range from 20,00 to 29,000.

13. The dialysis solution according to claims 11 and 12, characterized in that the molar substitution MS of the hydroxyethyl starch has a value in the range from 0.10 to 0.24.

14. The dialysis solution according to Claim 13, characterized in that the substitution MS of the hydroxyethyl starch has a value in the range from 0.20 to 0.24.

15. The dialysis solution according to Claims 11 to 14, characterized in that the substitution DS of the hydroxyethyl starch has a value in the range from 0.09 to 0.23.

16. The dialysis solution according to Claims 11 to 15, characterized in that the hydroxyethyl starch has a ratio C2/C6 in the range from 8 to 25.

17. The dialysis solution according to Claims 11 to 16, characterized in that the hydroxyethyl starch is present in a concentration in the range from 3 to 10 percent by weight, based on the final solution.

18. The dialysis solution according to Claims 11 to 17, characterized in that the dialysis solution contains from 3 to 10 percent by weight of hydroxyethyl starch, 125 to 150 mMol/litre of sodium ions, 90 to 115 mMol/litre of chloride ions, 0.3 to 1.5 mMol/litre of magnesium ions, 1.0 to 2.5 mMol/litre of calcium ions and 30 to 45 mMol/litre of lactate and/or acetate and/or bicarbonate.

19. The dialysis solution according to Claims 11 to 17, characterized in that the dialysis solution also contains, as additional ingredient, from 0.1 to 4.5 percent by weight of glucose, based on the final solution.

## Revendications

1. Utilisation d'hydroxyéthylamidon comme substance à activité osmotique dans une solution de dialyse pour la dialyse péritonéale, la solution contenant en plus de l'hydroxyéthylamidon des électrolytes et/ou des additifs classiques, caractérisée en ce que l'hydroxyéthylamidon a une masse moléculaire Mw de 10 000 à 150 000, un degré de substitution MS de 0,10 à 0,40, un degré de substitution DS de 0,09 à 0,35 et un rapport de substitution C2/C6 supérieur ou égal à 8.

2. Utilisation d'hydroxyéthylamidon selon la revendication 1, caractérisée en ce que l'hydroxyéthylamidon a une masse moléculaire Mw de 10 000 à 55 000.

3. Utilisation d'hydroxyéthylamidon selon la revendication 2, caractérisée en ce que l'hydroxyéthylamidon a une masse moléculaire de 20 000 à 29 000.

4. Utilisation d'hydroxyéthylamidon selon les revendications 1 à 3, caractérisée en ce que le degré de substitution molaire MS de l'hydroxyéthylamidon est compris entre 0,10 et 0,24.

5. Utilisation d'hydroxyéthylamidon selon la revendication 4, caractérisée en ce que le degré de substitution MS de l'hydroxyéthylamidon est compris entre 0,20 et 0,24.

6. Utilisation d'hydroxyéthylamidon selon les revendications 1 à 5, caractérisée en ce que l'hydroxyéthylamidon présente un degré de substitution DS compris entre 0,09 et 0,23.

7. Utilisation d'hydroxyéthylamidon selon les revendications 1 à 6, caractérisée en ce que l'hydroxyéthylamidon a un rapport de substitution C2/C6 compris entre 8 et 25.

8. Utilisation d'hydroxyéthylamidon selon les revendications 1 à 7, caractérisée en ce que l'hydroxyéthylamidon se trouve à une concentration de 3 à 10 % en masse par rapport à la solution finale.

9. Utilisation d'hydroxyéthylamidon selon les revendications 1 à 8, caractérisée en ce qu'elle contient 3 à 10 % en masse d'hydroxyéthylamidon, 125 à 150 mmol/l d'ions sodium, 90 à 115 mmol/l d'ions chlorure, 0,3 à 1,5 mmol/l d'ions magnésium, 1,0 à 2,5 mmol/l d'ions calcium et 30 à 45 mmol/l de lactate et/ou d'acétate et/ou de bicarbonate.

10. Utilisation d'hydroxyéthylamidon selon les revendications 1 à 9, caractérisée en ce qu'elle contient comme autre constituant 0,1 à 4,25 % en masse de glucose par rapport à la solution finale.

11. Solution de dialyse pour la dialyse péritonéale, contenant de l'hydroxyéthylamidon comme substance à activité osmotique, des électrolytes et/ou des additifs classiques, caractérisée en ce que l'hydroxyéthylamidon a une masse moléculaire Mw de 10 000 à 55 000, un degré de substitution MS de 0,10 à 0,40, un degré de substitution DS de 0,09 à 0,35 et un rapport de substitution C2/C6 supérieur ou égal à 8.

12. Solution de dialyse selon la revendication 11, caractérisée en ce que l'hydroxyéthylamidon a une masse moléculaire Mw de 20 000 à 29 000.

13. Solution de dialyse selon les revendications 11 et 12, caractérisée en ce que le degré de substitution molaire MS de l'hydroxyéthylamidon est compris entre 0,10 et 0,24.

14. Solution de dialyse selon la revendication 13, caractérisée en ce que le degré de substitution MS de l'hydroxyéthylamidon est compris entre 0,20 et 0,24.

15. Solution de dialyse selon les revendications 11 à 14, caractérisée en ce que l'hydroxyéthylamidon présente un degré de substitution DS compris entre 0,09 et 0,23.

16. Solution de dialyse selon les revendications 11 à 15, caractérisée en ce que l'hydroxyéthylamidon a un rapport de substitution C2/C6 compris entre 8 et 25.

17. Solution de dialyse selon les revendications 11 à 16, caractérisée en ce que l'hydroxyéthylamidon se trouve à une concentration de 3 à 10 % en masse par rapport à la solution finale.

18. Solution de dialyse selon les revendications 11 à 17, caractérisée en ce qu'elle contient 3 à 10 % en masse d'hydroxyéthylamidon, 125 à 150 mmol/l d'ions sodium, 90 à 115 mmol/l d'ions chlorure, 0,3 à 1,5 mmol/l d'ions magnésium, 1,0 à 2,5 mmol/l d'ions calcium et 30 à 45 mmol/l de lactate et/ou d'acétate et/ou de bicarbonate.

19. Solution de dialyse selon les revendications 11 à 18, caractérisée en ce qu'elle contient comme autre constituant 0,1 à 4,25 % en masse de glucose par rapport à la solution finale.
